# EUROPÄISCHE PATENTSCHRIFT

(11) **EP 0 537 540 B1**
(45) Veröffentlichungstag und Bekanntmachung des Hinweises auf die Patenterteilung: **23.10.1996**
(21) Anmeldenummer: 92116539.5
(22) Anmeldetag: 28.09.1992
(51) Int. Cl.: C07B 39/00

(54) **Verfahren zur radikalischen Chlorierung oder Bromierung von Methylaromaten**
Process for the radical chlorination or bromination of methyl substituted aromatics
Procédé de chloration radicalaire ou de bromuration radicalaire d'aromatiques méthyl substitués

(30) Priorität: 11.10.1991 DE 4133676
(43) Veröffentlichungstag der Anmeldung: 21.04.1993
(73) Patentinhaber: BAYER AG, 51368 Leverkusen (DE)
(72) Erfinder: Steffan, Guido, Dr., W-5068 Odenthal (DE)

(56) Entgegenhaltungen:
- EP-A- 0 306 804
- DE-A- 3 039 884
- US-A- 4 046 656

## Beschreibung

Die Erfindung betrifft ein radikalisches Verfahren zur Seitenkettenchlorierung oder -bromierung von Methylaromaten der weiter unten beschriebenen Art, bei denen gegebenenfalls vorhandene Sulfohalogenidgruppen unter Eliminierung von Schwefeldioxid in kernständiges Halogenid umgewandelt werden.

Die Seitenkettenhalogenierung von Methylaromaten, beispielsweise die Seitenkettenchlorierung zu den korrespondierenden Trichlormethyl-Aromaten, ist ebenso wie die Zersetzung aromatischer Sulfohalogenide, beispielsweise aromatischer Sulfochloride, zu den entsprechenden Arylhalogeniden gut bekannt (Houben-Weyl, Methoden der Org. Chemie, Band V/III S. 716). Beide Verfahren verlaufen nach einem radikalischen Mechanismus und werden in der chemischen Technik unter Belichtung, unter Zusatz von Radikalbildnern oder rein thermisch durchgeführt. Radikalbildner sind beispielsweise Phosphorpentachlorid, Sulfurylchlorid, Schwefelchloride, Iod, Peroxide und Azoverbindungen. Bei der Seitenkettenhalogenierung werden nacheinander, jedoch teilweise überlappend, die Benzylstufe, die Benzalstufe und schließlich die Benzotrihalogenidstufe erreicht. Insbesondere zur Erreichung der Benzotrihalogenidstufe werden längere Reaktionszeiten und Halogenüberschüsse benötigt; deswegen wird beispielsweise bei der kontinuierlich durchgeführten Toluolseitenkettenchlorierung das Chlor im Gegenstrom zum flüssigen Toluol geführt.

Auch die gleichzeitige Zersetzung einer Sulfochloridgruppe unter Eliminierung von SO₂ und die Chlorierung einer Methylgruppe zur Trichlormethylgruppe sind bereits bekannt (Herstellung von 2,4-Dichlorbenzotrichlorid aus 2-Chlor-toluol-4-sulfochlorid: DE-PS 234 290; Frdl 10, 116). Hierin sind aber weder Reaktionszeiten, noch Ausbeuten, Qualitäten oder Höhe des erforderlichen Chlorüberschusses angegeben. Auch zur Größe des Reaktionsansatzes wird nichts ausgesagt.

Die Herstellung von 4-Trichlormethyl-benzoylchlorid durch Chlorierung von 4-Methyl-benzoylchlorid unter Belichtung ist in J. Chem. Soc. (London) 121, 2212 bis 2214 beschrieben. In EP 306 804 wird die Herstellung der gleichen Verbindung durch 40-stündige Chlorierung unter Katalyse durch Di-lauroyl-peroxid mit mäßiger Ausbeute (90 %) beschrieben.

Die Herstellung von 2,3-Dichlor-6-trichlormethyl-chinoxalin durch eine 80 bis 110-stündige Chlorierung in einer großen Menge von o-Dichlorbenzol als Lösungsmittel ist in DE-OS 3 039 884 beschrieben. Das Produkt wird hierbei sowohl in mäßiger Qualität (93 %ig) als auch in mäßiger Ausbeute (92,5 %) erhalten,

Die Herstellung von 2,4-Dichlor-5-fluor-benzotrichlorid wird in DE-OS 3 142 856 beschrieben, Hierbei wird ein durch Diazotierung und nachfolgende Fluorierung von 3-Amino-4,6-dichlortoluol erhaltenes 2,4-Dichlor-5-fluortoluol unter Belichtung chloriert. Das Endprodukt wird in einer Ausbeute von 85 % der theoretischen Ausbeute erhalten; Reaktionszeiten sind hierbei nicht angegeben.

Die Benzotrihalogenide, insbesondere die Benzotrichloride, mit unterschiedlichen Substituenten werden vielfach als Vorprodukte benötigt, beispielsweise zur Hydrolyse zu den entsprechenden Carbonsäuren bzw. den Carbonsäurehalogeniden sowie für eine Reihe von Kondensatotionsreaktionen. Ihre Gewinnung durch radikalische Halogenierung, beispielsweise durch radikalische Chlorierung, ist bislang nur sehr unbefriedigend durchführbar, weil die Halogenierung, insbesondere beim Übergang von der Benzalstufe zur Benzotrihalogenidstufe, sehr viel Zeit in Anspruch nimmt und hohe Chlorüberschüsse verlangt (vgl. oben angeführte Einzelbeispiele). Versucht man, zur Verkürzung der Halogenierungszeit bei erhöhten Temperaturen zu arbeiten, wird ein merklicher Anteil der Trihalogenidgruppe abgespalten und dadurch Ausbeute und Gehalt des Endproduktes verringert. Außerdem beginnt bei drastisch verschärften Reaktionsbedingungen die Bildung kernhalogenierter Produkte, was die Qualität des Endproduktes weiterhin beeinträchtigt.

Während bei sehr kleinen Laboransätzen, etwa mit 0,5 mol Ausgangsmaterial, und bei Verwendung üblicher radikalischer Initiatoren, noch erträgliche Chlorierzeiten von etwa 15 bis 20 Stunden erzielt werden, steigen bei größeren Ansätzen im technischen Maßstab die notwendigen Chlorierzeiten drastisch an. Selbst die genannten Laboransätze benötigen hierbei allerdings Chlormengen im Bereich von 5,5 bis 8 mol pro mol Ausgangsmaterial, während der theoretische Wert 3 mol Chlor pro mol Ausgangsmaterial beträgt. Für den oben erwähnten Fall der gleichzeitigen radikalischen Eliminierung von SO₂ aus einer Sulfohalogenidgruppe neben der Seitenkettenhalogenierung bremst das entstehende SO₂ grundsätzlich die Halogenierung. Bei kleinen Laboransätzen gelingt die Austreibung des SO₂ durch überschüssigen Halogendampf; diese Austreibung funktioniert bei einer Ansatzvergrößerung immer schlechter. So wurde aufgrund der Laborergebnisse für eine Chlorierung in einem 6 m³-Chlorierreaktor eine reine Chlorierzeit von etwa 80 bis 100 Stunden abgeschätzt. Die Alternative, die in einer Verwendung extremer Chlorüberschüsse besteht, verbietet sich aus ökologischen Gründen und brächte auch nur eine graduelle Verbesserung.

Überraschenderweise wurd nun gefunden, daß die radikalische Seitenkettenhalogenierung durch den Zusatz eines oder mehrerer Halogenide der schweren Alkalimetalle in vorteilhafter Weise beeinflußt werden kann, Hierbei sind geringere Reaktionszeiten auch bei nur mäßigen Halogenüberschüssen erreichbar, Ausbeute und Qualität des Halogenierungsproduktes sind sehr gut.

Aus US 4 046 656 ist die Photochlorierung von Methylaromaten in Gegenwart von Brom zur Reaktionsbeschleunigung bekannt; Brom kann hierbei auch aus KBr und dem eingesetzten Cl₂ entwickelt werden. Das Brom dient der Erhöhung der UV-Absorption.

Die Erfindung betrifft demnach ein Verfahren zur radikalischen Chlorierung oder Bromierung von Methylaromaten der Formel (I) 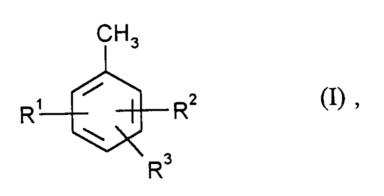 in der
- R¹ und R²: unabhängig voneinander Wasserstoff, Halogen, COHal oder SO₂Hal bedeuten und
- R³: Wasserstoff, Halogen oder COHal darstellt, wobei R² und R³ gemeinsam, wenn sie benachbart sind, den Rest eines 5- oder 6-gliedrigen isocyclischen oder heterocyclischen Ringes bilden können, der seinerseits 1- oder 2-fach durch Halogen, COHal oder SO₂Hal substituiert sein kann, wobei bei heterocyclischen Ringen diese 1 oder 2 Heteroatome aus der Gruppe von N, O und S enthalten,
zu Trichlormethyl- oder Tribrommethylaromaten der Formel (II) 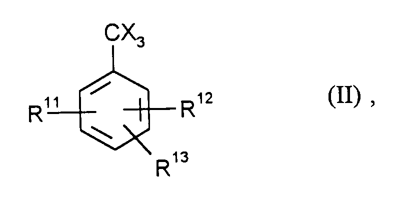
in der
- R¹¹ und R¹²: unabhängig voneinander Wasserstoff, Halogen oder COHal bedeuten,
- R¹³: Wasserstoff, Halogen oder COHal darstellt, wobei R¹² und R¹³, wenn sie benachbart sind, den Rest eines 5- oder 6-gliedrigen isocyclischen oder heterocyclischen Ringes bilden können, der seinerseits 1- oder 2-fach durch Halogen oder COHal substituiert sein kann, wobei bei heterocyclischen Ringen diese 1 oder 2 Heteroatome aus der Gruppe von N, O und S enthalten, und
- X: Chlor oder Brom darstellt,
und wobei im Falle des Vorliegens von SO₂Hal-Gruppen diese unter SO₂-Eliminierung in Halogen übergehen, das dadurch gekennzeichnet ist, daß die Halogenierung bei 120 bis 240°C unter Ausschluß von Radikalbildnern und unter Ausschluß einer photochemischen Initiierung in Gegenwart eines oder mehrerer Alkalihalogenide aus der Gruppe von KCl, KBr, RbCl, RbBr, CsCl und CsBr durchgeführt wird, wobei bei einer Chlorierung eines oder mehrere der genannten Alkalichloride und bei einer Bromierung eines oder mehrere der genannten Alkalibromide eingesetzt werden.

Zur Vermeidung eines unerwünschten Halogenaustausches wird bei einer Chlorierung eines oder mehrere der genannten Alkalimetallchloride und bei einer Bromierung eines oder mehrere der genannten Alkalimetallbromide einzusetzen. In bevorzugter Weise werden die genannten Halogenide von Kalium und von Caesium, besonders bevorzugt ein Gemisch des Kalium- und Caesiumhalogenids, bei einer Chlorierung also ein Gemisch aus KCl und CsCl eingesetzt. Die Menge des einzusetzenden Alkalimetallhalogenids beträgt 0,1 - 30 mMol pro Mol des zu halogenierenden Methyl-Aromaten.

Um ein Zusammensintern bzw. Zusammenballen der Alkalimetallhalogenide zu kugeligen Gebilden während der Halogenierung zu verhindern, hat es sich als vorteilhaft erwiesen, den Salzen einen inerten Feststoff zuzusetzen, der gewissermaßen die Funktion eines Trägers übernimmt. Solche inerten Feststoffe sind beispielsweise Celite. Die Menge dieses zugesetzen inerten Feststoffes beträgt ca. 50 - 200 % der Menge des verwendeten Alkalimetallhalogenids.

In weiterhin vorteilhafter Weise wird dem Reaktionsgemisch ein unter den Halogenierungsbedingungen hinreichend stabiles tertiäres Amin, beispielsweise Pyridin, in einer Menge von ca. 0,1 - 2 g, vorzugsweise ca. 0,5 g pro Mol Methylaromat zugesetzt. Dessen Wirkungsweise ist mit der eines Co-Katalysators zu vergleichen.

Halogen bzw. Hal in Formel (I) bedeuten unabhängig voneinander Fluor, Chlor oder Brom, bevorzugt Fluor oder Chlor.

Die zu halogenierenden Methylaromaten liegen im allgemeinen aus ihrer Herstellung in verunreinigter Form vor.

Solche Verunreinigungen sind beispielsweise aus der Einführung anderer funktioneller Gruppen Reste von Thionylchlorid oder Zersetzungsprodukte der unterschiedlichsten Art. Diese Verunreinigungen beeinträchtigen normalerweise die radikalische Halogenierung, so daß die Ausgangsstoffe bei herkömmlichen radikalischen Seitenkettenhalogenierungen vorher durch Destillation, Kristallisation, Filtration oder andere Maßnahmen gereinigt werden müssen, Es ist nun ein weiterer Vorteil des erfindungsgemäßen Verfahrens, daß eine solche Vorreinigung des Ausgangsmaterials nicht erforderlich ist.

In bevorzugter Weise wird das erfindungsgemäße Verfahren als Chlorierung durchgeführt, wobei in oben beschriebener Weise bevorzugt ein Gemisch aus KCl und CsCl eingesetzt wird.

In bevorzugter Weise wird als Methylaromat eine Verbindung der Formel (III) 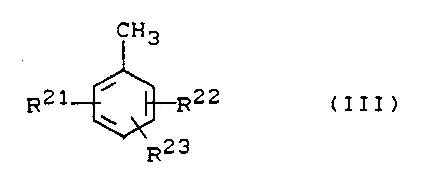 eingesetzt, in der
- R²¹ und R²²: unabhängig voneinander Wasserstoff, Fluor, Chlor, COF, COCl, SO₂F oder SO₂Cl bedeuten und
- R²³: Wasserstoff, F oder Cl bedeutet, wobei R²² und R²³ gemeinsam den Rest eines 6-gliedrigen aromatischen N-Heterocyclus bedeuten, der als zweites Heteroatom ein weiteres N-Atom enthalten kann, wobei der Heterocyclus 1- oder 2-fach durch Fluor oder Chlor substituiert sein kann.

In besonders bevorzugter Weise wird als Methylaromat eine Verbindung der Formel (IV) 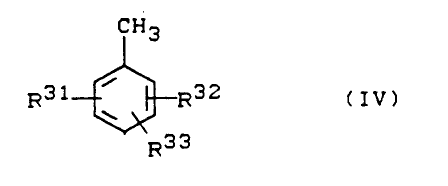 eingesetzt, in der
- R³¹ und R³²: unabhängig voneinander Wasserstoff, Chlor, COCl oder SO₂Cl bedeuten und
- R³³: Wasserstoff, Fluor oder Chlor darstellt, wobei R³² und R³³ gemeinsam den Rest des Chinoxalinringes bedeuten, der in 2- und/oder 3-Stellung durch Fluor oder Chlor substituiert sein kann.

Der Reaktionsverlauf wird anhand wichtiger Beispiele durch die folgenden Formelgleichungen verdeutlicht:

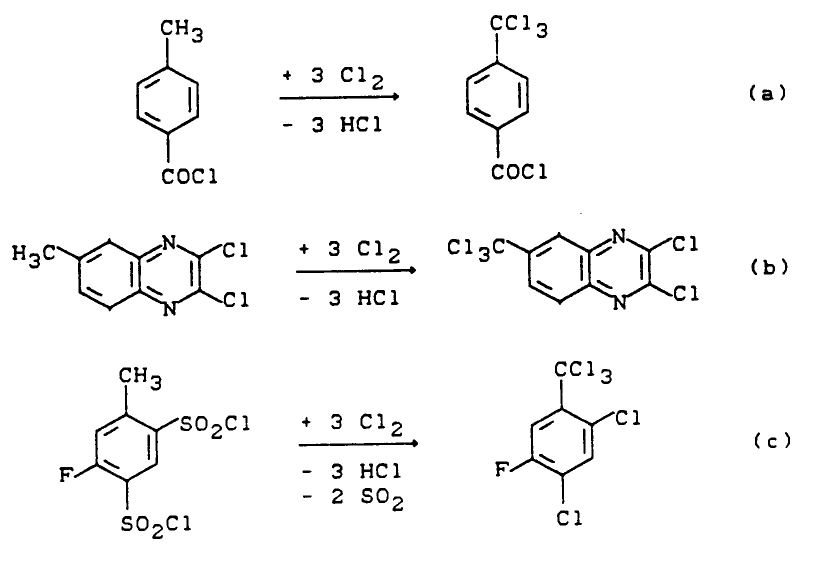

Das erfindungsgemäße Verfahren wird bei einer Temperatur von 120 - 240°C, bevorzugt von 160 - 220°C und einem Druck von 0,5 - 5 bar, bevorzugt von 1 - 1,5 bar, durchgeführt. Die Reaktionszeiten sind in einer dem Fachmann bekannten Weise von der Größe des Reaktionsansatzes abhängig, betragen aber im allgemeinen nur etwa 25 bis 85 % der Zeit, die ohne die erfindungsgemäße Katalyse nötig ist.

### Beispiele

### Beispiel 1

### 2,4-Dichlor-5-fluor-benzotrichlorid

970 g rohes 5-Fluortoluol-2,4-disulfochlorid, hergestellt nach der in EP 202 493 beschriebenen Methode (entsprechend 3,0 mol Reinprodukt bzw. 3,1 mol zur Disulfochlorid-Herstellung eingesetztem m-Fluortoluol), wurden in geschmolzenem Zustand versetzt mit
- 0,5 g: CsCl
- 4,5 g: KCl und
- 3 g: Celite.

Es wurden insgesamt 1 400 g Chlor in insgesamt 23 Stunden nach folgendem Schema eingeleitet:
- 400 g: Chlor bei 160 bis 180°C in 5 Stunden
- 800 g: Chlor bei 180 bis 197°C in 12 Stunden
- 200 g: Chlor bei 197 bis 195°C in 6 Stunden.

Nach Ende der Chlorierung enthielt das Rohprodukt nach Gaschromatogramm
- ca. 89 %: 2,4-Dichlor-5-fluor-benzotrichlorid
- ca. 1,8 %: 2,4-Dichlor-5-fluor-benzalchlorid
- ca. 3,1 %: 1,2,4-Trichlor-5-fluorbenzol
- ca. 2 %: diverse Spuren (nicht vollständig bestimmt)
(neben ca. 4 % separat bestimmten unverdampfbaren Anteilen).

Durch destillative Aufarbeitung wurden nach Abtrennen des Trichlorfluorbenzol-Vorlaufs
1. 15 g des o. g. Benzalchlorids erhalten (ca. 2 % d. Th.), die dem nächsten Ansatz zugeschlagen werden und
2. 768 g des erwünschten Benzotrichlorids mit folgender Zusammensetzung:
   - 0,5 %: 2,4-Dichlor-5-fluor-benzalchlorid
   - 98,2 %: 2,4-Dichlor-5-fluor-benzotrichlorid
   - 1,3 %: verschiedene Unbekannte.
   Dies entspricht einer Ausbeute von 89 % der theoretischen Ausbeute, die sich durch die o. g. Rückführung des Benzalchlorids in den Folgeansätzen auf ca. 91 % d. th. Ausbeute erhöht.

### Beispiel 1a (Vergleichsbeispiel, nicht beansprucht)

Es wurde das gleiche Edukt in gleicher Menge wie bei Beispiel 1 eingesetzt. Statt CsCl/KCl/Celite wurden 19 g PCl₅ als Katalysator verwendet (zugesetzt in mehreren Portionen). Wegen der deutlich langsameren Chloraufnahme wurden insgesamt 2 945 g Chlor in insgesamt 48 Stunden nach folgendem Schema eingeleitet:
- 600 g: Chlor bei 170 bis 195°C in 10 Stunden
- 1 200 g: Chlor bei 195 bis 200°C in 26 Stunden
- 245 g: Chlor bei 198 bis 200°C in 12 Stunden.

Nach Aufarbeitung des Reaktionsgemisches (gemäß Gaschromatogramm:
- 2,2 %: 2,4-Dichlor-5-fluor-benzalchlorid
- 86,5 %: 2,4-Dichlor-5-fluor-benzotrichlorid
- 3,2 %: 1,2,4-Trichlor-5-fluorbenzol
- ca. 4 %: diverse Spuren, nicht vollständig bestimmt
neben ca. 4 % separat bestimmten unverdampfbaren Anteilen)
wurden - wie in Beispiel 1 beschrieben -
- ca. 86 %: d. th. Ausbeute des o. g. Benzotrichlorids
- und 2,5 %: d. th. Ausbeute des o. g. Benzalchlorids
erhalten.

Bei Rückführung des Benzalchlorids in die Folgeansätze wurde eine Gesamtausbeute von ca. 88,5 % d. th. Ausbeute an o. g. Benzotrichlorid erhalten.

### Beispiel 2

### 2,4-Dichlor-5-fluor-benzotrichlorid (technisches Beispiel)

In 5 860 kg im Reaktor befindliche Chlorsulfonsäure wurden im Laufe von 8 Stunden 2 750 kg m-Fluortoluol eindosiert, wobei die Temperatur unter HCl-Entwicklung auf 70°C stieg; danach wurde in weiteren 2 Stunden auf 90°C aufgeheizt.

Im Laufe von 4 Stunden wurden nun ca. 1 000 kg Chlorsulfonsäure-Thionylchlorid-Destillat aus einer Vorpartie eindosiert (darin ca. 600 kg Chlorsulfonsäure und 400 kg Thionylchlorid); anschließend wurden weiterhin bei 90°C 4 100 kg Thionylchlorid im Laufe von 18 Stunden eindosiert; danach wurde in 3 Stunden auf 155°C aufgeheizt, diese Temperatur 2 Stunden gehalten, dann auf 120°C abgekühlt.

Bei dieser Temperatur wurden weitere 2 200 kg Thionylchlorid in 9 Stunden eindosiert.

Nach einer Nachreaktionszeit von 3 Stunden wurden ca. 1 000 kg überschüssiges Chlorsulfonsäure-Thionylchlorid-Gemisch im Vakuum bis zu einer Sumpftemperatur von 160°C abdestilliert und bei der Folgepartie - wie oben beschrieben - wieder eingesetzt. Man erhielt etwa 7 650 kg rohes 5-Fluortoluol-2,4-disulfochlorid, das ohne weitere Nachbehandlung in die Chlorierung eingesetzt wurde. (Die Ausbeute, bezogen auf m-Fluortoluol, betrug etwa 97 bis 98 % d. th. Ausbeute).

Es wurden 10 kg KCl, 7 kg Celite und 0,15 kg CsCl zugesetzt, auf 165°C geheizt und dann insgesamt 7 200 kg Chlor nach folgendem Schema eingeleitet:
- 4 800 kg: Chlor bei 165 bis 195°C in 14 Stunden
- 1 600 kg: Chlor bei 195 bis 210°C in 7 Stunden
- 800 kg: Chlor bei 210 bis 205°C in 8 Stunden.

Vor Einleitung der letzten 800 kg Chlor wurde der Zwischenlauf aus der Destillation der Vorpartie (ca. 800 kg mit einem Gehalt von 30 bis 40 % Dichlorfluorbenzalchlorid) der Partie zugesetzt.

(Es ist darauf zu achten, daß bei Erreichen von 180°C bei der o. g. Chlorierung wenigstens 1 400 kg Chlor bereits eingeleitet sind, weil sonst das Reaktionsgemisch zur Zersetzung neigt. Mit zunehmendem Chlorgehalt verschwindet diese Zersetzlichkeit allmählich.)

Bei der nachfolgenden destillativen Aufarbeitung wurden zuerst ca. 130 kg (2,5 % d. th. Ausbeute) Trichlorfluorbenzol als Vorlauf erhalten, dann 800 kg Zwischenlauf (mit einem Gehalt von 30 bis 40 % Dichlorfluorbenzalchlorid) und schließlich ca. 6 685 kg Dichlorfluorbenzotrichlorid mit folgender Zusammensetzung:
- 99,3 %: 2,4-Dichlor-5-fluor-benzotrichlorid
- 0,2 - 0,3 %: 2,4-Dichlor-5-fluor-benzalchlorid
- 0,2 %: isomeres Dichlorfluor-benzalchlorid und diverse Spuren.

Dies entsprach einer Ausbeute von ca. 94 % d. th. Ausbeute, bezogen auf m-Fluortoluol bzw. einer Ausbeute von ca. 96 % d. th.Ausbeute, bezogen auf das 5-Fluortoluol-2,4-disulfochlorid.

Als Destillationsrückstand blieben ca. 200 kg zurück.

Ohne Zusatz von Caesiumchlorid macht sich besonders bei der Chlorierung im technischen Maßstab die Bildung eines hochschmelzenden Sublimat-Belags in Brüdenrohr und Kondensator sehr störend bemerkbar.

### Beispiel 3

### 4-Trichlormethyl-benzoylchlorid

4-Methylbenzoylchlorid wurde in üblicher Weise aus 4-Methylbenzoesäure und Thionylchlorid hergestellt und nach Abdestillieren überschüssigen Thionylchlorids als Rohprodukt in die Seitenkettenchlorierung eingesetzt.

1 097 g rohes 4-Methylbenzoylchlorid, 99,85 %ig, (entspr. 7,0 Mol) wurden mit
- 2 g: Caesiumchlorid und
- 3,5 g: Pyridin versetzt und nach folgendem Schema chloriert:
Unter intensivem Rühren wurden eingeleitet
- 1 200 g: Chlor bei 195 bis 200°C in 14 Stunden
- 450 g: Chlor bei 200 bis 210°C in 5 Stunden
- 250 g: Chlor bei 210 bis 215°C in 5 Stunden.

Man erhielt 1 798 g Chlorierprodukt mit einem Gehalt von 98,2 % (nach Gaschromatogramm) 4-Trichlormethyl-benzoylchlorid. Nach Vakuumdestillation wurden 1 763 g Produkt mit einem Gehalt von 99,2 % 4-Trichlormethyl-benzoylchlorid (nach Gaschromatogramm) erhalten, was einer Ausbeute von 96,9 % d. th. Ausbeute entspricht.

### Beispiel 3a (Vergleichsbeispiel, nicht beansprucht)

Eine in gleicher Weise wie im Beispiel 3, aber ohne Zusatz von Caesiumchlorid durchgeführte Chlorierung benötigte 28 Stunden Chlorierzeit und ergab 1 788 g Rohprodukt mit einem Gehalt von 95,4 % 4-Trichlormethyl-benzoylchlorid (nach Gaschromatogramm).

Nach Vakuumdestillation wurden 1 754 g Produkt mit einem Gehalt von 96,8 % 4-Trichlormethyl-benzoylchlorid (nach Gaschromatogramm) erhalten, was einer Ausbeute von 94 % d. th. Ausbeute entspricht. (Das Produkt enthielt in geringer Menge offenbar kernchlorierte Nebenprodukte.)

### Beispiel 4

### 2,3-Dichlor-6-trichlormethyl-chinoxalin

2,3-Dichlor-6-methyl-chinoxalin wurde durch Kondensation von Oxalsäure mit 3,4-Diamino-toluol und nachfolgende Umsetzung mit Thionylchlorid hergestellt und als Rohprodukt (nach Abdestillieren überschüssigen Thionylchlorids) in die Chlorierungen eingesetzt.
- 880 g: 96,1 %ige Rohware (3,97 mol) wurden mit
- 2 g: KCl
- 1 g: CsCl
- 2 g: Celite
- 2 g: Pyridin und
- 30 g: Chlorbenzol (zur Vermeidung von Sublimat-Anbackung im Kondensator)
versetzt und nach folgendem Schema chloriert:
Unter intensivem Rühren wurden eingeleitet
- 870 g: Chlor bei 135 bis 160°C in 9 Stunden
- 280 g: Chlor bei 160 bis 190°C in 2,5 Stunden
- 150 g: Chlor bei 190 bis 200°C in 2,5 Stunden.

Das Gaschromatogramm des Rohprodukts (1 334 g) zeigte einen Gehalt von
- 95,3 %: 2,3-Dichlor-6-trichlormethyl-chinoxalin und
- 0,3 %: 2,3-Dichlor-6-dichlormethyl-chinoxalin
neben einigen Höhersiedern (Summe 3,5 %) und ca. 1 % separat bestimmten unverdampfbaren Anteilen.

Nach Vakuumdestillation wurden 1 252 g Destillat mit einem Gehalt von 96,8 % an 2,3-Dichlor-6-trichlormethyl-chinoxalin erhalten, was einer Ausbeute von 98,5 % d. th. Ausbeute entspricht.

### Beispiel 4a (Vergleichsbeispiel, nicht beansprucht)

Die Chlorierung wurde in gleicher Weise durchgeführt, wie im Beispiel 4 beschrieben, jedoch wurden als Katalysator nur 2 g Pyridin zugesetzt und kein CsCl, KCl und Celite.

Sie beansprucht eine Zeit von 20 Stunden (statt 14 Stunden) und benötigte insgesamt 1 580 g Chlor (statt 1.300 g).

Erhalten wurden 1 325 g Rohprodukt mit einem Gehalt (nach Gaschromatogramm) von
- 90,2 %: 2,3-Dichlor-6-trichlormethyl-chinoxalin
- 0,8 %: 2,3-Dichlor-6-dichlormethyl-chinoxalin
neben mehreren Höhersiedern (Summe: 7,1 %) und ca. 2 % separat bestimmten unverdampfbaren Anteilen.

Nach Vakuumdestillation wurden 1 255 g Destillat mit einem Gehalt von 93,8 % an 2,3-Dichlor-6-trichlormethyl-chinoxalin erhalten, was einer Ausbeute von 93,7 % d. th. Ausbeute entspricht.

## Patentansprüche

1. Verfahren zur radikalischen Chlorierung oder Bromierung von Methylaromaten der Formel 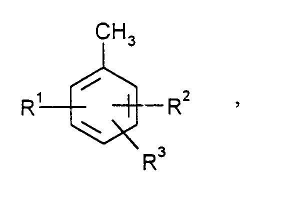 in der
R¹ und R² unabhängig voneinander Wasserstoff, Halogen, COHal oder SO₂Hal bedeuten und
R³ Wasserstoff oder Halogen bedeutet, wobei R² und R³ gemeinsam, wenn sie benachbart sind, den Rest eines 5- oder 6-gliedrigen isocyclischen oder heterocyclischen Ringes bilden können, der seinerseits 1- oder 2-fach durch Halogen, COHal oder SO₂Hal substituiert sein kann, wobei bei heterocyclischen Ringen diese 1 oder 2 Heteroatome aus der Gruppe von N, O und S enthalten,
zu Trichlormethyl- oder Tribrommethylaromaten der Formel 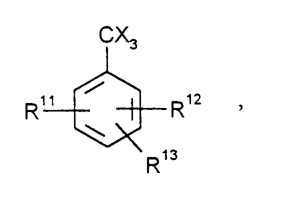
in der
R¹¹ und R¹² unabhängig voneinander Wasserstoff, Halogen oder COHal bedeuten,
R¹³ Wasserstoff, Halogen oder COHal darstellt, wobei R¹² und R¹³, wenn sie benachbart sind, den Rest eines 5- oder 6-gliedrigen isocyclischen oder heterocyclischen Ringes bilden können, der seinerseits 1- oder 2-fach durch Halogen oder COHal substituiert sein kann, wobei bei heterocyclischen Ringen diese 1 oder 2 Heteroatome aus der Gruppe von H, O und S enthalten, und
X Chlor oder Brom darstellt,
und wobei im Falle des Vorliegens von SO₂Hal-Gruppen diese unter SO₂-Eliminierung in Halogen übergehen,
dadurch gekennzeichnet, daß die Halogenierung bei 120 bis 240°C unter Ausschluß von Radikalbildnern und unter Ausschluß einer photochemischen Initiierung in Gegenwart eines oder mehrerer Alkalihalogenide aus der Gruppe KCl, KBr, RbCl, RbBr, CsCl und CsBr durchgeführt wird, wobei bei einer Chlorierung eines oder mehrere der genannten Alkalichloride und bei einer Bromierung eines oder mehrere der genannten Alkalibromide eingesetzt werden.

2. Verfahren nach Anspruch 1, dadurch gekennzeichnet, daß eine Chlorierung der Methylgruppe durchgeführt wird,.

3. Verfahren nach Anspruch 2, dadurch gekennzeichnet, daß ein Gemisch aus KCl und CsCl eingesetzt wird.

4. Verfahren nach Anspruch 1, dadurch gekennzeichnet, daß als Methylaromat eine Verbindung der Formel 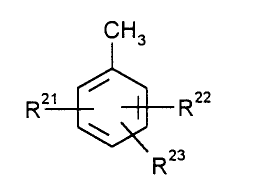 eingesetzt wird, in der
R²¹ und R²² unabhängig voneinander Wasserstoff, Fluor, Chlor, COF, COCl, SO₂F oder SO₂Cl bedeuten und
R²³ Wasserstoff, Fluor oder Chlor darstellt, wobei R²² und R²³ gemeinsam den Rest eines 6-gliedrigen aromatischen N-Heterocyclus bedeuten, der als zweites Heteroatom ein weiteres N-Atom enthalten kann.

5. Verfahren nach Anspruch 4, dadurch gekennzeichnet, daß als Methylaromat eine Verbindung der Formel 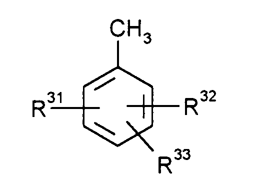 eingesetzt wird, in der
R³¹ und R³² unabhängig voneinander Wasserstoff, Chlor, COCl oder SO₂Cl bedeuten und
R³³ Wasserstoff, Fluor oder Chlor darstellt, wobei R³² und R³³ gemeinsam den Rest des Chinoxalinringes bedeuten, der in 2- und/oder 3-Stellung durch Fluor oder Chlor substituiert sein kann.

6. Verfahren nach Anspruch 1, dadurch gekennzeichnet, daß eines oder mehrere Alkalimetallhalogenide gemeinsam mit einem inerten Feststoff eingesetzt werden.

7. Verfahren nach Anspruch 1, dadurch gekennzeichnet, daß eines oder mehrere der Alkalimetallhalogenide gemeinsam mit einem Amin eingesetzt werden.

8. Verfahren nach Anspruch 1, dadurch gekennzeichnet, daß der zu chlorierende oder bomierende Methylaromat in Form eines Rohproduktes aus seiner Herstellung ohne vorherige Reinigung eingesetzt wird.

## Claims

1. Process for the free-radical chlorination or bromination of methylaromatic compounds of the formula 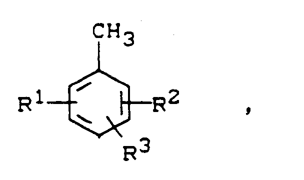 in which
R¹ and R² independently of each other denote hydrogen, halogen, COHal or SO₂Hal and
R³ denotes hydrogen or halogen, where R² and R³ together, if they are adjacent, can form the radical of a 5- or 6-membered isocyclic or heterocyclic ring, which can itself be monosubstituted or disubstituted by halogen, COHal or SO₂Hal, where such heterocyclic rings contain 1 or 2 hetero atoms selected from the group consisting of N, O and S,
to give trichloromethyl aromatic compounds or tribromomethyl aromatic compounds of the formula 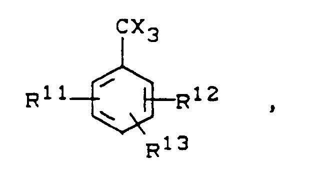
in which
R¹¹ and R¹² independently of each other denote hydrogen, halogen or COHal,
R¹³ is hydrogen, halogen or COHal, where R¹² and R¹³, if they are adjacent, can form the radical of a 5- or 6-membered isocyclic or heterocyclic ring, which itself can be monosubstituted or disubstituted by halogen or COHal, where such heterocyclic rings contain 1 or 2 hetero atoms selected from the group consisting of N, O and S, and
X is chlorine or bromine,
and where, in the case of the occurrence of SO₂Hal groups, these are converted to halogen with elimination of SO₂,
characterised in that the halogenation is carried out at 120 to 240°C with the exclusion of free-radical formers and with the exclusion of a photochemical initiation in the presence of one or more alkali metal halides selected from the group consisting of KCl, KBr, RbCl, RbBr, CsCl and CsBr, in the case of a chlorination, one or more of the said alkali metal chlorides being used, and in the case of a bromination one or more of the said alkali metal bromides being used.

2. Process according to Claim 1, characterised in that a chlorination of the methyl group is carried out.

3. Process according to Claim 2, characterised in that a mixture of KCl and CsCl is used.

4. Process according to Claim 1, characterised in that the methylaromatic compound used is a compound of the formula 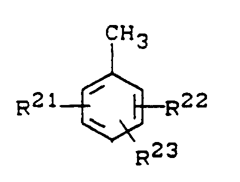 in which
R²¹ and R²² independently of each other denote hydrogen, fluorine, chlorine, COF, COCl, SO₂F or SO₂Cl and
R²³ is hydrogen, fluorine or chlorine, where R²² and R²³ together denote the radical of a 6-membered aromatic N-heterocycle, which can contain a further N-atom as second hetero atom.

5. Process according to Claim 4, characterised in that the methylaromatic compound used is a compound of the formula 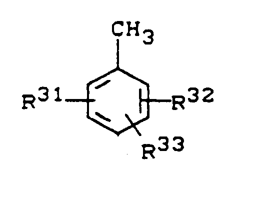 in which
R³¹ and R³² independently of each other denote hydrogen, chlorine, COCl or SO₂Cl and
R³³ is hydrogen, fluorine or chlorine, where R³² and R³³ together denote the radical of the quinoxaline ring, which can be substituted in the 2- and/or 3-position by fluorine or chlorine.

6. Process according to Claim 1, characterised in that one or more alkali metal halides are used together with an inert solid.

7. Process according to Claim 1, characterised in that one or more of the alkali metal halides are used together with an amine.

8. Process according to Claim 1, characterised in that the methylaromatic compound to be chlorinated or brominated is used in the form of a crude product from its preparation without previous purification.

## Revendications

1. Procédé pour la chloruration ou la bromuration radicalaire de dérivés méthylaromatiques de formule 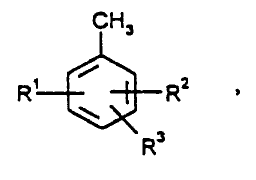 dans laquelle
R¹ et R² représentent chacun, indépendamment l'un de l'autre, l'hydrogène, un halogène, un groupe COHal ou SO₂Hal et
R³ représente l'hydrogène ou un halogène, R² et R³ pouvant également former ensemble, lorsqu'ils sont voisins, le radical d'un noyau isocyclique ou hétérocyclique à cinq ou six chaînons qui peut lui-même porter un ou deux substituants halogéno, COHal ou SO₂Hal et, dans le cas d'un noyau hétérocyclique, contient un ou deux hétéroatomes choisis parmi N, O et S,
en dérivés trichlorométhyl- ou tribromométhyl-aromatiques de formule 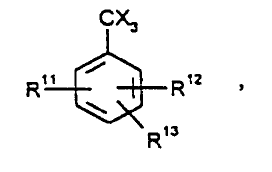
dans laquelle
R¹¹ et R¹² représentent chacun, indépendamment l'un de l'autre, l'hydrogène, un halogène ou un groupe COHal,
R¹³ représente l'hydrogène, un halogène ou un groupe COHal, R¹² et R¹³ pouvant également former ensemble, lorsqu'ils sont voisins, le radical d'un noyau isocyclique ou hétérocyclique à cinq ou six chaînons qui peut lui-même porter un ou deux substituants halogéno ou COHal et, dans le cas d'un noyau hétérocyclique, contient un ou deux hétéroatomes choisis parmi N, O et S, et
X représente le chlore ou le brome,
avec, dans le cas de groupes SOHal présents, conversion de ces groupes en halogènes par élimination de SO₂,
caractérisé en ce que l'on réalise l'halogénation à des températures de 120 à 240°C, en l'absence de formateurs de radicaux libres et en l'absence d'une induction photochimique, en présence d'un ou plusieurs halogénures alcalins du groupe formé par KCl, KBr, RbCl, RbBr, CsCl et CsBr, avec mise en oeuvre d'un ou plusieurs des chlorures alcalins en question dans le cas d'une chloruration et d'un ou plusieurs des bromures alcalins en question dans le cas d'une bromuration.

2. Procédé selon la revendication 1, caractérisé en ce que l'on procède à une chloruration du groupe méthyle.

3. Procédé selon la revendication 2, caractérisé en ce que l'on utilise un mélange de KCl et de CsCl.

4. Procédé selon la revendication 1, caractérisé en ce que l'on met en oeuvre en tant que dérivé méthylaromatique un composé de formule 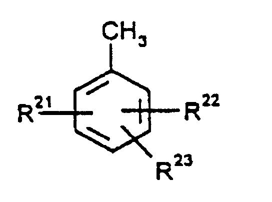 dans laquelle
R²¹ et R²² représentent chacun, indépendamment l'un de l'autre, l'hydrogène, le fluor, le chlore, un groupe COF, COCl, SO₂F ou SO₂Cl et
R²³ représente l'hydrogène, le fluor ou le chlore, R²² et R²³ pouvant également former ensemble le radical d'un hétérocycle aromatique azoté à six chaînons qui peut contenir, en tant que deuxième hétéroatome, un autre atome d'azote.

5. Procédé selon la revendication 4, caractérisé en ce que l'on met en oeuvre en tant que dérivé méthylaromatique un composé de formule 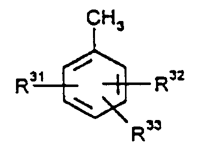 dans laquelle
R³¹ et R³² représentent chacun, indépendamment l'un de l'autre, l'hydrogène, le chlore, un groupe COCl ou SO₂Cl et
R³³ représente l'hydrogène, le fluor ou le chlore, R³² et R³³ pouvant également former ensemble le radical du noyau quinoxaline qui peut être substitué en position 2 et/ou 3 par le fluor ou le chlore.

6. Procédé selon la revendication 1, caractérisé en ce que l'on met en oeuvre un ou plusieurs halogénures de métaux alcalins avec une matière solide inerte.

7. Procédé selon la revendication 1, caractérisé en ce que l'on met en oeuvre un ou plusieurs des halogénures de métaux alcalins avec une amine.

8. Procédé selon la revendication 1, caractérisé en ce que le dérivé méthylaromatique à chlorer ou bromer est mis en oeuvre à l'état de produit brut de sa préparation, sans purification préalable.
